# EUROPEAN PATENT SPECIFICATION

(11) **EP 1 815 827 B1**
(45) Date of publication and mention of the grant of the patent: **15.09.2010**
(21) Application number: 07250437.6
(22) Date of filing: 02.02.2007
(51) Int. Cl.: A61F 5/00

(54) **Gastric band introduction device**
Einführvorrichtung für Magenband
Dispositif d'introduction de bandelette gastrique

(30) Priority: 03.02.2006 US 346592
(43) Date of publication of application: 08.08.2007
(73) Proprietor: ETHICON ENDO-SURGERY, INC., Cincinnati, Ohio 45242 (US)
(72) Inventor: Tsonton, Mark, Loveland, OH 45140 (US)
(74) Representative: Tunstall, Christopher Stephen

(56) References cited:
- EP-A- 1 520 563
- EP-A1- 0 364 420
- EP-A1- 0 556 056
- US-A1- 2002 022 851

## Description

### BACKGROUND OF THE INVENTION

### 1. Field of the Invention

The present invention relates to an introduction device for gastric bands. In particular, the invention relates to an introduction device including a transparent distal end permitting viewing of the gastric band housed therein.

### 2. Description of the Prior Art

Morbid obesity is a serious medical condition. In fact, morbid obesity has become highly pervasive in the United States, as well as other countries, and the trend appears to be heading in a negative direction. Complications associated with morbid obesity include hypertension, diabetes, coronary artery disease, stroke, congestive heart failure, multiple orthopedic problems and pulmonary insufficiency with markedly decreased life expectancy. With this in mind, and as those skilled in the art will certainly appreciate, the monetary and physical costs associated with morbid obesity are substantial. In fact, it is estimated the costs relating to obesity are in excess of 100 billion dollars in the United States alone.

A variety of surgical procedures have been developed to treat obesity. The most common currently performed procedure is Roux-en-Y gastric bypass (RYGB). This procedure is highly complex and is commonly utilized to treat people exhibiting morbid obesity. Other forms of bariatric surgery include Fobi pouch, bilio-pancreatic diversion, and gastroplastic or "stomach stapling". In addition, implantable devices are known which limit the passage of food through the stomach and affect satiety.

In view of the highly invasive nature of many of these procedures, efforts have been made to develop less traumatic and less invasive procedures. Gastric-banding is one such procedure. Gastric-banding is a type of gastric reduction surgery attempting to limit food intake by reducing the size of the stomach. In contrast to RYGB and other stomach reduction procedures, gastric-banding does not require the alteration of the anatomy of the digestive tract in the duodenum or jejunum.

Since the early 1980s, gastric bands have provided an effective alternative to gastric bypass and other irreversible surgical weight loss treatments for the morbidly obese. Several alternative procedures are performed under the heading of gastric-banding. Some banding techniques employ a gastric ring, others use a band, some use stomach staples and still other procedures use a combination of rings, bands and staples. Among the procedures most commonly performed are lap band, vertical banded gastroplasty (VBG), silastic ring gastroplasty (SRG), and adjustable silastic gastric banding (AGB).

In general, the gastric band is wrapped around an upper portion of the patient's stomach, forming a stoma that is less than the normal interior diameter of the stomach. This restricts food passing from an upper portion to a lower digestive portion of the stomach. When the stoma is of an appropriate size, food held in the upper portion of the stomach provides a feeling of fullness that discourages overeating.

Typically, the gastric band is laparoscopically introduced into a patient's abdomen by pushing it through a trocar. Since inserting the gastric band directly through a trocar may also undesirably damage the gastric band, delivery devices have been developed wherein the gastric band is preloaded with the delivery device and is ready for implantation without worrying that the gastric band may contact an exterior skin surface or become damaged as it is moved through the trocar.

European publication no. EP1 520 563 discloses a surgical tool for implanting a gastric band onto a patient's abdomen, including an inner rod disposed inside a support tube. The preamble of claim 1 is based on this document.

Despite the many advantages associated with delivery instruments employing a preloaded gastric band, the recent development of balloon-type gastric bands necessitates various checks prior to insertion of the delivery instrument, for example, it is now often considered desirable to leak test balloon-type gastric bands prior to insertion into the abdominal cavity for implantation. As such, a need currently exists for improved gastric band delivery systems providing for convenient and reliable delivery of gastric bands, including techniques permitting testing of the gastric bands prior to implantation. The present invention provides such a gastric band delivery technique.

### SUMMARY OF THE INVENTION

The present invention therefore provides a gastric band introduction device, comprising: an elongated support tube having a handle at a proximal end thereof and an opening for delivery of a gastric band at a distal end thereof; and a transparent section formed at the distal end of the support tube.

The distal end may include a transparent distal tube member.

The support tube may be co-extruded or molded.

The transparent section may extend only partially about a circumference of the support tube.

The gastric band introduction device may further include an inner rod having a proximal end and a distal end, the inner rod is located within the support tube and adapted to slide therein.

The inner rod may include a thumb ring at a proximal end and one or more upwardly protruding pins for engaging a gastric band at the distal end thereof.

The inner rod may further include a shelf located at the distal end thereof for holding the gastric band.

The inner rod may further include a longitudinal protrusion for effecting the sliding engagement between the inner rod and the support tube and also for stabilizing the inner rod when it is inserted into the support tube.

The gastric band introduction device may further include a gastric band and a tether secured to the gastric band for controlling deployment thereof.

A method for the pre-application inspection of a gastric band through the utilization of a gastric band introduction device, comprising: removing the gastric band introduction device from its packaging; and initiating a check of the gastric band prior to introduction within the patient, the check including viewing of the gastric band through a transparent section formed in the introduction device.

The gastric band may be a balloon type gastric band and initiation of a check includes performing a leak test of the balloon-type gastric band.

The leak test may include applying air pressure to the balloon-type gastric band and observing through the transparent section of the support tube whether the balloon inflates in a desired manner.

The leak test may include evacuating the gastric band prior to loading into the introduction device.

Objects and advantages of the present invention will become apparent from the following detailed description when viewed in conjunction with the accompanying drawings, which set forth certain embodiments of the invention.

### BRIEF DESCRIPTION OF THE DRAWINGS

Figure 1 is a perspective view of an unactuated gastric band introduction device in accordance with the present invention.
Figure 2 is a perspective view of an actuated gastric band introduction device as shown in Figure 1.
Figure 3 is a partial side view of the distal end of the inner rod of the gastric band introduction device.
Figure 4 is a perspective view of an actuated gastric band introduction device with the gastric band partially removed from the inner rod.
Figure 5 is an enlarged partial view of the inner rod with the gastric band partially removed.
Figure 6 is a perspective view of an alternate embodiment of a gastric band introduction device.

### DESCRIPTION OF THE PREFERRED EMBODIMENTS

The detailed embodiments of the present invention are disclosed herein. It should be understood, however, that the disclosed embodiments are merely exemplary of the invention, which may be embodied in various forms. Therefore, the details disclosed herein are not to be interpreted as limiting, but merely as the basis for the claims and as a basis for teaching one skilled in the art how to make and/or use the invention.

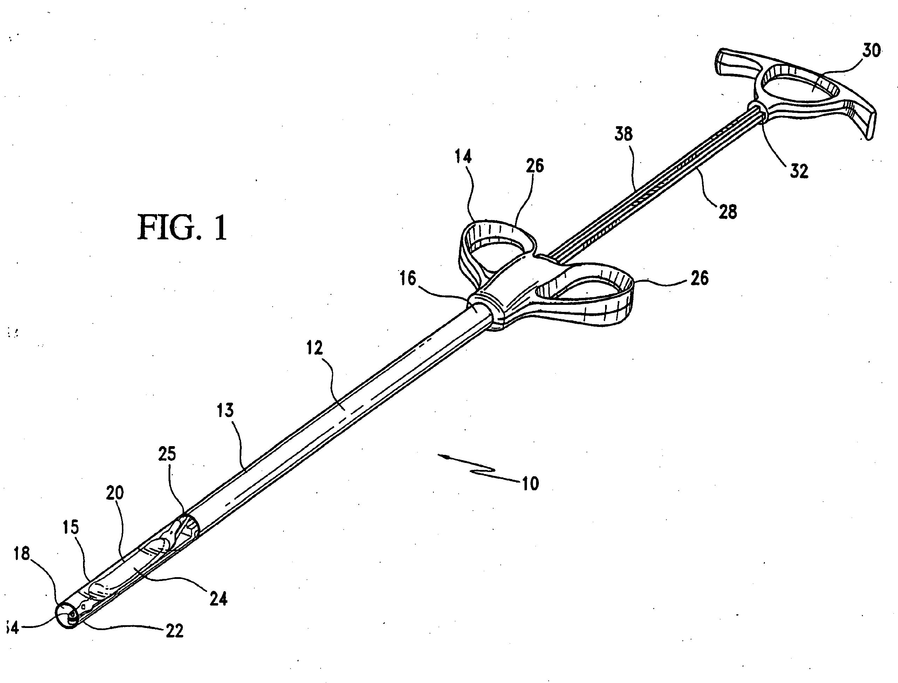

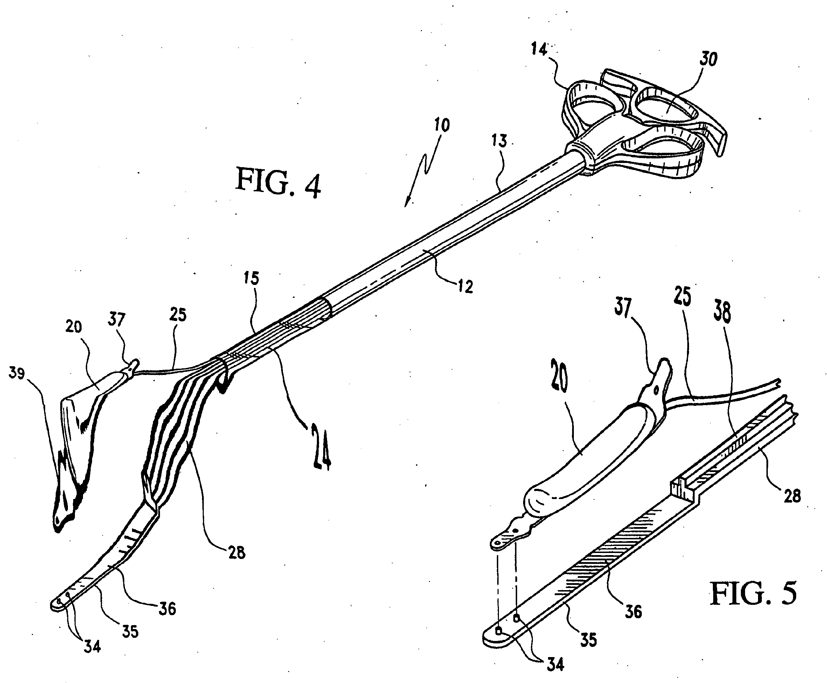

Referring to the Figures 1 to 5, a gastric band introduction device 10 is shown. The gastric band introduction device 10 includes an elongated support tube 12 having a handle 14 at a proximal end 16 thereof and an opening 18 for delivery of a gastric band 20 at a distal end 22 thereof. As will be discussed below in greater detail, the distal end 22 of the support tube 12 is provided with a transparent section 24 allowing viewing of the gastric band 20 stored therein. The gastric band 20 is held in position by a tether 25 extending up through the support tube 12 for control by the medical practitioner introducing the gastric band 20.

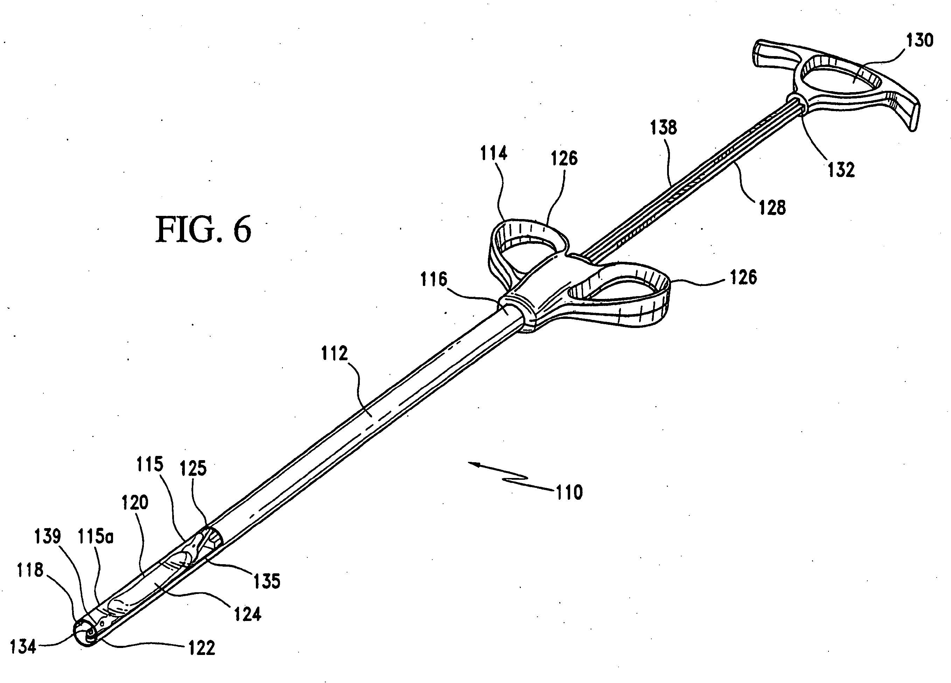

It is contemplated the transparent section may be formed in a variety of ways. For example, and with reference to Figures 1 to 5, the support tube 12 may be formed from a proximal tube member 13 and a distal tube member 15 joined to define the complete support tube 12. The proximal tube member 13 may be formed from a variety of materials, while the distal tube member 15 is preferably a clear extruded tube. In accordance with this embodiment and as those skilled in the art will certainly appreciate, the joining of the proximal tube member 13 and the distal tube member 15 may be accomplished by a snap joint, glue joint or weld joint. Similarly the support tube could be co-extruded or molded with the transparent portion of the tube positioned at the distal end. In addition, and reference to Figure 6, the distal tube member 115 could be formed with only a portion 115a thereof being transparent (that is, half of the tube cross-section like a door). For example, the distal end 122 of the support tube 112 would be provided with an arcuate transparent section 115a that extends only partially about the circumference of the support tube 112.

Referring to Figure 1, an isometric view of an unactuated gastric band introduction device 10 ready for introduction into a patient is presented. The gastric band introduction device 10 includes an elongated support tube 12 with a handle 14 at a proximal end 16. The gastric band introduction device 10 preferably includes a pair of opposed finger loops 26 extending outwardly from the handle 14. An inner rod 28 is located to slide within the support tube 12. The inner rod 28 includes a thumb ring 30 at a proximal end 32 thereof and one or more upwardly protruding pins 34 for engaging the gastric band 20 at a distal end 35 thereof. The inner rod 28 preferably includes a shelf 36 located at the distal end 35 of the inner rod 28 for holding the gastric band 20. The inner rod 28 also includes a longitudinal protrusion 38 shaped and dimensioned for engaging a longitudinal groove (not shown) in the support tube 12 for effecting the sliding engagement between the inner rod 28 and support tube 12. The provision of the longitudinal protrusion and the longitudinal groove also function to stabilize the inner rod 28 when it is inserted into the support tube 12.

The support tube 12 is preferably comprised of a durable engineered plastic, although other materials may be used. Preferably, the support tube 12 has a diameter of about 10 mm to about 20 mm and more preferably between about 12 mm and about 15 mm. Preferably, the support tube 12 is between about 30 cm and about 50 cm in length and more preferably about 43 cm. However, it should be appreciated that the support tube can be adapted in both length and diameter to accommodate any kind of gastric band or to fit within any sized trocar. The handle, the finger loops, the inner rod, and the thumb ring can be comprised of either stainless steel or any suitably durable engineering plastic. While pins are shown in the illustrative embodiment, any other mechanism, such as a clip or a strap, which will releasably secure the gastric band to the inner rod while also allowing the gastric band to be easily removed from the inner rod will suffice.

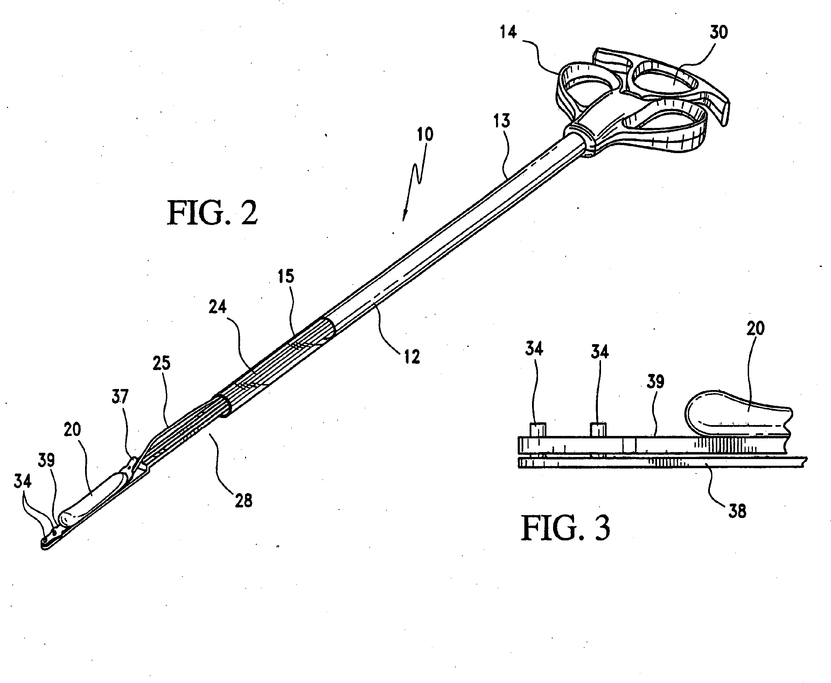

Figures 2 through 5 show the gastric band introduction device 10 after the device has been actuated to deploy a gastric band 20 from the distal end 22 of the support tube 12. In Figures 2 through 5, the inner rod 28 has been fully inserted into the support tube 12 and has pushed the gastric band 20 from the distal end 22 of the support tube 12. Once the gastric band 20 has been deployed, the gastric band 20 can be removed from the inner rod 28.

Generally, the gastric band 20 is delivered to a desired location via the utilization of the tether 25 to control the proximal end 37 of the gastric band 20 while the distal end 35 of the gastric band 20 is manipulated via a control arm releasably attached via clamp to the distal end 39 of the gastric band 20 for wrapping the gastric band about an individual's stomach in a well known manner.

In use, the medical practitioner removes the gastric band introduction device 10 from its packaging and initiates a check of the gastric band 20 prior to implantation within the patient. In particular, the medical practitioner will perform a leak test of the balloon-type gastric band 20. The leak testing procedure involves applying air pressure to the balloon-type gastric band 20 and observing whether the balloon inflates in a desired manner. Because of the transparent section 24 at the distal end 22 of the support tube 12, the medical practitioner need only look through the support tube 12 as the leak test is performed. It is contemplated that leak testing may be further enhanced by evacuating the gastric band 20 prior to loading into the support tube 12. As such, the medical practitioner will be instructed to look for partially inflation of the gastric band's bladder, which would be indicative of a leak allowing for the passage of air into the gastric band's bladder.

Once testing is completed, the gastric band introduction device 10, in an unactuated state, is inserted into a trocar placed in the patient's abdomen. The device 10 is then actuated by sliding and inserting the inner rod 28 fully into the support tube 12, thereby deploying the gastric band 20. The gastric band 20 is then removed from the inner rod 28 for placement around the stomach. Once the gastric band 20 has been removed, the inner rod 28 is pulled back out of the support tube 12, and the device is removed from the trocar.

In accordance with an alternate embodiment as shown in Figure 6 and briefly discussed above, an alternate introduction device 110 may be utilized in delivering the gastric band 120 to a desired location. More particularly, the introduction device 110 includes an elongated support tube 112. As with the prior embodiment, the elongated support tube 112 includes a handle 114 at a proximal end 116 thereof and an opening 118 for delivery of a gastric band 120 at a distal end 122 thereof. The gastric band introduction device 110 also includes a pair of opposed finger loops 126 extending outwardly from the handle 114. An inner rod 128 is located to slide within the support tube 112. The inner rod 128 includes a thumb ring 130 at a proximal end 132 thereof and one or more upwardly protruding pins 134 for engaging the distal end 139 of the gastric band 120. The inner rod 128 preferably includes a shelf located at the distal end 135 of the inner rod 128 for holding the gastric band 120. The inner rod 128 also includes a longitudinal protrusion 138 shaped and dimensioned for engaging a longitudinal groove (not shown) in the support tube 112 for effecting the sliding engagement between the inner rod 128 and support tube 112. The provision of the longitudinal protrusion and the longitudinal groove also function to stabilize the inner rod 128 when it is inserted into the support tube 112.

As with the prior embodiment, the distal end 122 of the support tube 112 is provided with a transparent section 124 allowing viewing of the gastric band 120 stored therein. The gastric band 120 is positioned at the distal end 122 of the support tube 112 and is presented for viewing based upon the transparent section 124 at the distal end 122 thereof. However, and in accordance with the embodiment, the transparent section 124 only constitutes a portion of the arc defined by the support tube 112 at the distal end 124 thereof. More particularly, the distal end 122 of the support tube 112 is provided with an arcuate transparent section 115a that extends only partially about the circumference of the support tube 112. The gastric band 120 is held in position by a tether 125 extending up through the support tube 112 for control by the medical practitioner introducing the gastric band 120.

As with the prior embodiment, the support tube 112 is preferably comprised of a durable engineered plastic, although other materials may be used. The dimensions of the support tube 112 are similar to those discussed above and may be varied to accommodate any kind of gastric band or to fit within any size trocar.

In use, this embodiment is employed in a manner substantially similar to the embodiment disclosed with reference to Figures 1 to 5 and described above in detail.

It will become readily apparent to those skilled in the art that the above invention has equally applicability to other types of implantable bands. For example, bands are used for the treatment of fecal incontinence. One such band is described in U.S. Pat. No. 6,461,292. Bands can also be used to treat urinary incontinence. One such band is described in U.S. Patent Application 2003/0105385 incorporated herein by reference. Bands can also be used to treat heartburn and/or acid reflux. One such band is described in U.S. Pat. No. 6,470,892. Bands can also be used to treat impotence. One such band is described in U.S. Patent Application 2003/0114729.

While the present invention has been illustrated by the description of several embodiments and while the illustrative embodiments have been described in considerable detail, it is not the intention of the applicant to restrict or in any way limit the scope of the appended claims to such detail. Additional advantages and modifications may readily appear to those skilled in the art.

While the preferred embodiments have been shown and described, it will be understood that there is no intent to limit the invention by such disclosure, but rather, is intended to cover all modifications and alternate constructions falling within the scope of the invention.

## Claims

1. A gastric band introduction device (10), comprising:
a gastric band having a distal end and a proximal end to which a tether is secured;
an elongated support tube (12) having the gastric band stored therein having a handle (14) at a proximal end (16) thereof and an opening (18) for delivery of the gastric band (20) at a distal end (22) thereof; and
an inner rod having a proximal end and a distal end, the inner rod being located within the elongated support tube and adapted to slide therein, the distal end of the inner rod holding the gastric band; and **characterized by**,
a transparent section (24) formed at the distal end of the support tube.

2. The gastric band introduction device according to claim 1, wherein the distal end includes a transparent distal tube member (15).

3. The gastric band introduction device according to claim 1, wherein the support tube is co-extruded or molded.

4. The gastric band introduction device according to claim 1, wherein the transparent section extends only partially about a circumference of the support tube.

5. The gastric band introduction device according to claim 1, wherein the inner rod includes a thumb ring (30) at a proximal end and one or more upwardly protruding pins (34) for engaging a gastric band at the distal end thereof.

6. The gastric band introduction device according to claim 5, wherein the inner rod further includes a shelf (36) located at the distal end thereof for holding the gastric band.

7. The gastric band introduction device according to claim 6, wherein the inner rod further includes a longitudinal protrusion (38) for effecting the sliding engagement between the inner rod and the support tube and also for stabilizing the inner rod when it is inserted into the support tube.

8. The gastric band introduction device according to claim 1, wherein the tether (25) is secured to the gastric band for controlling deployment thereof.

9. A method for the pre-application inspection of a gastric band (20) through the utilization of a gastric band introduction device (10) having a gastric band stored therein, comprising:
removing the gastric band introduction device from its packaging; and
initiating a check of the gastric band prior to introduction within the patient, the check including viewing of the gastric band through a transparent section (24) formed in the introduction device.

## Patentansprüche

1. Vorrichtung (10) zum Einführen eines Magenbandes, die aufweist:
ein Magenband mit einem distalen Ende und einem proximalen Ende, an dem ein Halteelement gesichert ist;
ein längliches Stützrohr (12), in dem das Magenband untergebracht ist, mit einem Griff (14) an seinem proximalen Ende (16) und einer Öffnung (18) zum Zuführen des Magenbandes (20) an einem distalen Ende (22); und
eine innere Stange mit einem proximalen Ende und einem distalen Ende, wobei sich die innere Stange innerhalb des Stützrohres befindet und so ausgelegt ist, dass es darin gleitet, wobei das distale Ende der inneren Stange das Magenband hält; und **gekennzeichnet durch**
einen transparenten Abschnitt (24), der an dem distalen Ende des Stützrohres gebildet ist.

2. Vorrichtung zum Einführen eines Magenbandes nach Anspruch 1, bei der das distale Ende ein transparentes distales Rohrelement (15) aufweist.

3. Vorrichtung zum Einführen eines Magenbandes nach Anspruch 1, bei der das Stützrohr koextrudiert oder geformt ist.

4. Vorrichtung zum Einführen eines Magenbandes nach Anspruch 1, bei der sich der transparente Abschnitt nur teilsweise um einen Umfang des Stützrohres erstreckt.

5. Vorrichtung zum Einführen eines Magenbandes nach Anspruch 1, bei der die innere Stange eine Daumenöse (30) an einem proximalen Ende und einen oder mehrere nach oben hervorstehende Stifte (34) zum Greifen eines Magenbandes an dessen distalen Ende umfasst.

6. Vorrichtung zum Einführen eines Magenbandes nach Anspruch 5, bei der die innere Stange weiter ein Bord (36) zum Halten des Magenbandes umfasst, das sich an deren distalen Ende befindet.

7. Vorrichtung zum Einführen eines Magenbandes nach Anspruch 6, bei der die innere Stange weiter einen längs verlaufenden Vorsprung (38) zum Bewirken des Schiebeeingriffs zwischen der inneren Stange und dem Stützrohr und außerdem zum Stabilisieren der inneren Stange, wenn sie in das Stützrohr eingeführt wird, umfasst.

8. Vorrichtung zum Einführen eines Magenbandes nach Anspruch 1, bei der das Halteelement (25) an dem Magenband zum Steuern seines Anbringens gesichert ist.

9. Verfahren zum Überprüfen eines Magenbandes (20) vor seinem Anbringen durch die Verwendung einer Vorrichtung (10) zum Einführen eines Magenbandes, in der ein Magenband untergebracht ist, das aufweist:
Entnehmen der Vorrichtung zum Einführen eines Magenbandes aus ihrer Verpackung; und
Einleiten einer Prüfung des Magenbandes vor dem Einführen in den Patienten, wobei die Prüfung das Besichtigen des Magenbandes durch einen transparenten Abschnitt (24), der in der Einführvorrichtung gebildet ist, umfasst.

## Revendications

1. Dispositif d'introducticn de bandelette gastrique (10), comprenant :
■ une bandelette gastrique ayant une extrémité distale et une extrémité proximale à laquelle une attache est fixée ;
■ un tube de support allongé (12) ayant la bandelette gastrique stockée à l'intérieur de ce dernier, ayant une poignée (14) au niveau de son extrémité proximale (16) et une ouverture (18) pour la pose de ladite bandelette gastrique (20) au niveau de son extrémité distale (22) ; et une tige interne ayant une extrémité proximale et une extrémité distale, la tige interne étant positionnée à l'intérieur du tube de support allongé et adaptée pour coulisser à l'intérieur de ce dernier, l'extrémité distale de la tige interne maintenant la bandelette gastrique ; et **caractérisé par** :
■ une section transparente (24) formée au niveau de l'extrémité distale du tube de support.

2. Dispositif d'introduction de bandelette gastrique selon la revendication 1, dans lequel l'extrémité distale comprend un élément de tube distal transparent (15).

3. Dispositif d'introduction de bandelette gastrique selon la revendication 1, dans lequel le tube de support est co-extrudé ou moulé.

4. Dispositif d'introduction de bandelette gastrique selon la revendication 1, dans lequel la section transparente s'étend uniquement partiellement autour d'une circonférence du tube de support.

5. Dispositif d'introduction de bandelette gastrique selon la revendication 1, dans lequel la tige interne comprend un anneau de pouce (30) au niveau d'une extrémité proximale et une ou plusieurs broches en saillie vers le haut (34) pour mettre en prise une bandelette gastrique au niveau de son extrémité distale.

6. Dispositif d'introduction de bandelette gastrique selon la revendication 5, dans lequel la tige interne comprend en outre une plaque (36) positionnée au niveau de son extrémité distale pour maintenir la bandelette gastrique.

7. Dispositif d'introduction de bandelette gastrique selon la revendication 6, dans lequel la tige interne comprend en outre une saillie longitudinale (38) pour effectuer la mise en prise coulissante entre la tige interne et le tube de support et également pour stabiliser la tige interne lorsqu'elle est insérée dans le tube de support.

8. Dispositif d'introduction de bandelette gastrique selon la revendication 1, dans lequel l'attache (25) est fixée sur la bandelette gastrique pour contrôler son déploiement.

9. Procédé pour contrôler l'application préalable d'une bandelette gastrique (20) par le biais de l'utilisation d'un dispositif d'introduction de bandelette gastrique (10) ayant une bandelette gastrique stockée à l'intérieur de ce dernier, comprenant les étapes consistant à :
■ retirer le dispositif d'introduction de bandelette gastrique de son emballage ; et
■ initier un contrôle de la bandelette gastrique avant l'introduction dans le patient, le contrôle comprenant l'étape consistant à observer la bandelette gastrique à travers une section transparente (24) formée dans le dispositif d'introduction.
